# EUROPEAN PATENT SPECIFICATION

(11) **EP 2 700 371 B1**
(45) Date of publication and mention of the grant of the patent: **31.07.2019**
(21) Application number: 12773503.3
(22) Date of filing: 19.04.2012
(51) Int. Cl.: A61B 17/74, B22F 3/11, A61L 31/02, A61L 31/14

(54) **POROUS TANTALUM ROD**
PORÖSE TANTALSTANGE
TIGE POREUSE EN TANTALE

(30) Priority: 20.04.2011 CN 201110099357
(43) Date of publication of application: 26.02.2014
(73) Proprietor: Chongqing Runzer Pharmaceutical CO., LTD, Chongqing 401120 (CN)
(72) Inventor: YE, Lei, Yubei, Chongqing 401120 (CN)
(74) Representative: Plougmann Vingtoft a/s
(86) International application number: PCT/CN2012/074345
(87) International publication number: WO 2012/142952

(56) References cited:
- EP-A1- 1 344 537
- WO-A1-96/39974
- CN-A- 101 212 990
- CN-A- 101 660 076
- CN-A- 101 721 241
- CN-A- 101 721 241
- CN-A- 102 028 566
- CN-A- 102 205 144
- CN-U- 201 500 170
- CN-U- 202 060 915
- RU-C1- 2 076 647
- US-A1- 2003 153 981
- US-A1- 2005 048 193
- JIE, YUNFENG.: 'Research on the Preparation of Porous Tantalum and Its Process and Property.' THESIS OF MASTER DEGREE OF CENTRAL SOUTH UNIVERSITY. 15 January 2012, pages 19 - 32, XP008171233
- JIE, YUNFENG ET AL.: 'Effect of Sintering Temperature on Mechanical Properties and Microstructure of Nb Foams.' THE CHINESE JOURNAL OF NONFERROUS METALS. vol. 20, no. 10, October 2010, pages 2014 - 2018, XP008171224

## Description

### Field of invention

The present invention relates to a medical implantation, especially relates to a porous tantalum rod used for treating collapsed articular surface of the femoral head or necrosis of the femoral head in phase I or phase II.

### Description of the Related Art

Collapsed articular surface of the femoral head and necrosis of the femoral head are common clinical diseases. Currently, the drilling method for decompression is usually used for treating collapsed articular surface of the femoral head or necrosis of the femoral head. In the surgery, the sequestrum needs to be removed first, and then the medical implantation is filled for supporting collapse or necrosis of femoral head.

A porous metal material used for medical implantation is important for specific application of treating traumatic osseous tissues, necrotic femoral tissues or the like. Such metal materials are normally porous stainless steel, porous titanium, and so on. As a porous implant material for the treatment of traumatic osseous tissues and necrotic femoral tissues, the porosity thereof should reach to 30-85%, and the pores should be all interconnected and well-distributed or partially interconnected depending on requirement and well-distributed. Thus, the porous implant material can have the same growth phase with the osseous tissue and have lower weight for use of medical implantation.

Due to good biocompatibility and mechanical properties of the refractory tantalum, the porous form thereof is expected to take place of the traditional metal biomaterials mentioned above to be used as a medical implant material for treating necrotic femoral tissues. Also, due to the harmlessness, non-toxicity, none side effect, the rapid development of the medicine, and the further knowledge of tantalum as an implant material, the requirement of porous tantalum for medical implantation is getting more and more urgent, and the criterion for porous tantalum is getting higher and higher. As a porous tantalum for medical implantation, having a lot of well-distributed interconnecting pores and mechanical properties adaptable to human body are of great importance for being a connecting component to keep the newborn tissues growing well at the positions of traumatic osseous tissues or ossature defects.

Regarding porous tantalum, US Patent No. 5282861 discloses "Open cell tantalum structures for cancellous bone implants and cell and tissue receptors". The porous tantalum is made of pure commercial tantalum and takes a carbon skeleton obtained from heat degradation of polyurethane precursors as a supporter. The carbon skeleton has multiple dodecahedrons with mesh structures inside and wholly distributed pores, and the porosity thereof reaches to 98%. Next, the commercial tantalum is bound to the carbon skeleton to form porous metal microstructure through chemical vapor deposition (CVD) (also called "chemical deposition"). The porous tantalum material obtained by such processes has a tantalum layer having 40-60µm of thickness, and the whole porous tantalum materials has about 99 wt% of tantalum and about 1 wt% of carbon skeleton. The patent further discloses that the porous tantalum has 50-70MPa of compressive strength, 2.5-3.5GPa of elastic modulus, 63MPa of tensile strength and 15% of the amount of plastic deformation. However, the mechanical property such as ductility of the porous tantalum described above is obviously insufficient, causing the difficulty of subsequent processing of the porous tantalum, such as cutting and drilling the formed material. Currently, the medical implantation made of porous tantalum material used for filling collapsed articular surface of the femoral head or necrosis of the femoral head is a rod-like body with screw thread formed on one end of the rod-like body. People in the industry is accustomed to call it as a porous tantalum rod. When being used for implanting into the femoral head in clinical treatment, the porous tantalum rod relies on the natural growth of the femoral head to be confined and fixed. The porous tantalum rod with this type of structure is difficult to position precisely in surgery. In the surgery, the screwed structure of the porous tantalum rod is held by special surgical tool and screwed into the femoral head, and causing the rear end of screw fixed in the cortical bone of the femoral head to decrease, which results in the instability and dislocation the porous tantalum rod, , and non continuous depressurize. Overall, the medical implantation with this type of structure has lower success rate in surgery. Similar rods were defined in WO96/39974 A1.

### SUMMARY OF THE INVENTION

The objective of the present invention is to provide a porous tantalum rod used for medical implantation, which is easy to be positioned precisely and has good mechanical properties.

To achieve the objective, a porous tantalum rod is provided as defined in the claims.

The porous tantalum rod having the sintered neck structure formed between the tantalum particles greatly improves the mechanical properties of such implantation like ductility, anti-bending strength, and is easy to drill. The through hole in the center of the porous tantalum rod provides the convenience of precisely positioning in the surgery and convenience to remove and inject correlate, and achieves effect of decompression continually.

Preferably, the fastening structure of the porous tantalum rod is a screwed structure, and had a slot formed on the end-face of the screwed structure for installing and using surgical tool.

Preferably, the other end of the porous tantalum rod opposite to the fastening structure is ball head shaped.

The shape of the through hole in the center of the porous tantalum rod is circular-shaped, square-shaped or other shapes in cross section.

The porous tantalum rod is made of porous tantalum material made by foam impregnation. Specifically, the method for preparing the porous tantalum material comprising steps of:
(a) providing an organic binder, dispersant and tantalum powder;
(b) mixing the organic binder and the dispersant to form a solution and then mixing the solution and the tantalum powder to form tantalum slurry;
(c) providing an organic foam body, wherein the organic foam body has multiple pores;
(d) casting the tantalum slurry into the organic foam body and impregnating the casted organic foam body with the tantalum slurry until the pores of the organic foam body are filled with the tantalum slurry;
(e) drying the impregnated organic foam body with the tantalum slurry to remove the dispersant;
(f) degreasing the dried organic foam body to separate the dried tantalum slurry from the organic binder and the organic foam body in a protective environment of inert gas;
(g) vacuum sintering the dried tantalum slurry to obtain a porous sintered body, wherein the porous sintered body has a foam skeleton, sintered tantalum particles located on the foam skeleton, and multiple sintering neck structures formed between the tantalum particles; and
(h) vacuum annealing and treating the porous sintered body with normal post-treatments to obtain the porous tantalum.

The porous tantalum material made of the method maintains the mechanical properties of the porous tantalum with such sintered neck structure, as well as the improved ductility thereof. Also, the porous tantalum material prepared by such method can be conveniently and effectively used for the application of surgery implantation of medical metal material. The porous tantalum rod made of the method is specially suitable to be a medical implantation used for treating collapsed articular surface of the femoral head or necrosis of the femoral head in phase I or phase II. Furthermore, the method is simple and easy to control, and the processes of the method are harmless, non-polluting, non-toxic, non- dust, no side effects on the human body.

The porous tantalum material is produced by sintering the tantalum powder having an average diameter of less than 50µm and oxygen content of less than 0.1%, wherein the produced porous tantalum material has 30-85% of porosity and 150-600µm of pore diameter, and the sintering neck structures are formed between at least 50%-95% of the tantalum particles.

In the method for preparing the porous tantalum material, the organic foam body in the foam impregnation is a polyurethane foam body having 0.48-0.89mm of pore diameter, 0.015-0.065g/cm³ of density and larger than 40° of hardness.

In summary, the porous tantalum rod used for medical implantation in accordance with the present invention is including a through hole in the center of the porous tantalum rod. Therefore, the implantation can be passed through the through hole and screwed along the positioning pin (guide pin). After the implantation screwed, the guide pin can be removed easily from the through hole.

Mashed osseous tissues can be injected into the femoral head via a feeder through the through hole, thus the cavity of the femoral head without lesion osseous tissues is filled with good osseous tissues, so that the osseous tissues become more denser for the convenience of fixation of the implantation and growth of osseous tissues.

The medical implantation made of the porous tantalum material of the present invention is able to be long-term preserved in human body, has good support intensity and heals and fixes better with surrounding osseous tissues. More importantly, the through hole formed on the center of the porous tantalum rod is good for continuous depressurization during and after the operation. Overall, the porous tantalum rod of the present invention provides higher success rate in the implant surgery.

### BRIEF DESCRIPTION OF THE DRAWINGS

Fig. 1 is a schematic view of an embodiment of a porous tantalum rod in accordance with the present invention;
Fig. 2 is a left view of a porous tantalum rod in Fig. 1;
Fig. 3 is a right view of a porous tantalum rod in Fig. 1;
Fig. 4 is a schematic view of a porous tantalum rod being positioned by using guide pin in accordance with the present invention;
Fig. 5 is a schematic view of a porous tantalum coordinated with feeder in accordance with the present invention;
Fig. 6 is a x-ray diffraction (XRD) pattern of a porous tantalum material used for a porous tantalum rod in accordance with the present invention;
Fig. 7 is a scanning electron microscopic (SEM) macrograph of a porous tantalum material used for a porous tantalum rod in accordance with the present invention;
Fig. 8 is a scanning electron microscopic (SEM) micrograph of a porous tantalum material used for a porous tantalum rod in accordance with the present invention.

### DETAILED DESCRIPTION OF THE INVENTION

### Example 1:

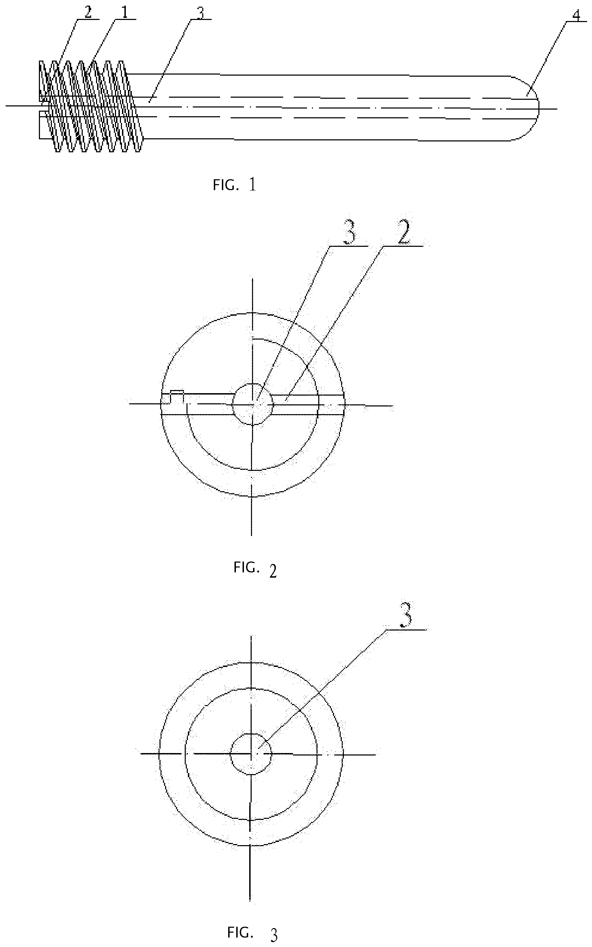

Referring to Figs. 1 to 3, a porous tantalum rod in accordance with the present invention comprises: a fastening structure 1 with screw thread formed on one end of the porous tantalum rod used for connecting with osseous tissues; a ball head shaped structure 4 formed on the other end of the porous tantalum rod; as shown in Figs. 2, a slot 2 formed on the end-face of the fastening structure 1 for installing and using the surgical tools; and a through hole 3 formed on the center of the porous tantalum rod as shown in Fig. 3, wherein the cross section of the through hole 3 is circular-shaped in this example, but can also be square-shaped or other shapes that is easy to position and remove and inject correlate object. The porous tantalum material of the porous tantalum rod has a foam structure with three-dimensional interconnecting pores, which is produced by foam impregnation. The foam structure has a foam skeleton, and sintered tantalum particles accumulated on the foam skeleton, and multiple sintering neck structures formed between the tantalum particles.

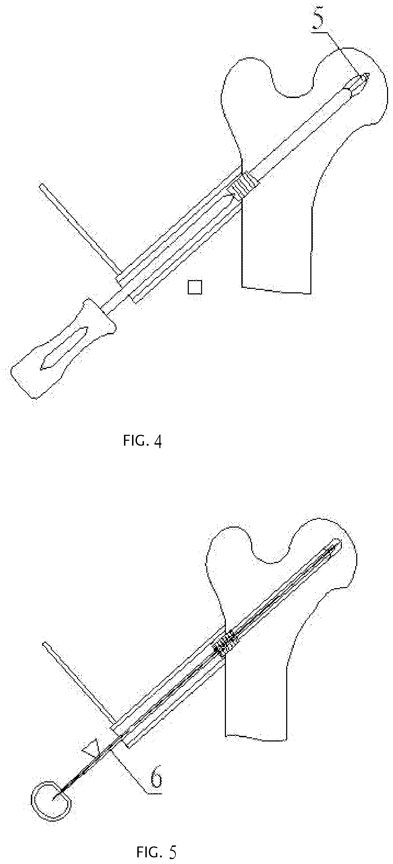

Referring to Fig. 4, due to the through hole is in the center of the porous tantalum rod, the implantation is able to be screwed along the guide pin (positioning pin) in surgery. In middle of the implantation process, screwdriver rotates the implantation via the slot 2 formed on the end-face of the fastening structure. Preferably, the implantation is a screwed fastening structure, thus the installation of the implantation can be positioned in position precisely.

Further referring Fig.5, the guide pin 5 is able to be removed from the through hole 3 of the implantation when the porous tantalum rod is in middle of implantation as shown in Fig. 4. Mashed osseous tissues can be injected towards ball head shaped structure 4 via a feeder 6 through the through hole, thus the cavity of the femoral head without lesion osseous tissues is filled with good osseous tissues, so that the osseous tissues become more denser for the convenience of fixation of the implantation and growth of osseous tissues.

### Example 2:

The structure of porous tantalum rod is illustrated in Example 1. In this example, a method for preparing the porous tantalum material of the porous tantalum rod is provided.

The solution of 2-8 wt% polyvinyl alcohol solution made by polyvinyl alcohol and distilled water is mixed with tantalum powder having less than 50µm of diameter and less than 0.1% of the oxygen content to form tantalum slurry. The tantalum slurry was casted into the polyurethane foam having 0.48-0.89 mm of pore diameter, 0.015-0.065g/cm³ of density and hardness larger than 40°until the organic foam body is filled with the tantalum slurry. The polyurethane foam body with the tantalum slurry was dried to remove water therein, degreased under inert gas to remove polyvinyl alcohol and polyurethane foam, and vacuum sintered to form a porous sintered body. The foam skeleton formed by the sintered tantalum power has tantalum particles with 50%-90% of sintered neck structures, and then, the foam skeleton is vacuum annealed and treated with normal post-treatments to obtain a porous tantalum.

### Example 3:

A method for preparing the porous tantalum material of the porous tantalum rod is provided.

12.5g of polyvinyl alcohol was put in a container filled in 240ml of water, and then the container was put on a hotplate. The polyvinyl alcohol and water are heated and mixed to form a polyvinyl alcohol solution. 60g of tantalum powder with less than 50µm of diameter and less than 0.1% of oxygen content weighted by a 300g scale is added to 50ml of the polyvinyl alcohol solution (the polyvinyl alcohol solution was cooled). The tantalum powder and the polyvinyl alcohol solution were mix and agitated homogeneously to form tantalum slurry. The tantalum slurry was casted into a 10×10×30mm porous polyurethane foam body (0.48mm of pore diameter, 0.025g/cm³ of density and 50° of hardness) until the pores of the polyurethane foam body were filled with the tantalum slurry. Then, the polyurethane foam body filled with the tantalum slurry was put into a porcelain dish placed in a vacuum drier. The polyurethane foam body filled with the tantalum slurry was dried in the vacuum drier at 60°C for 8 hours under 1Pa of vacuity. The dried polyurethane foam body filled with the tantalum slurry was degreased at 600°C for 120 minutes under lower than 10⁻⁴Pa of vacuity. Then, the dried tantalum slurry are sintered in a vacuum sintering furnace at 2200°C for 2 hours under 10⁻⁴Pa of vacuity to form a porous sintered body. The argon is employed as a protective gas during sintering. The porous sintered body was cleaned to remove the dust and dirt and then treated with normal post-treatments to obtain a porous tantalum.

Produce a rod with the above-mentioned porous tantalum material, and the rod comprises a screw thread formed on one end of rod used for connecting with osseous tissues; a ball head shaped structure formed on the other end of the rod; a slot formed on the end-face of the end with screw thread for installing and using the surgical tools; a through hole formed on the center of the rod.

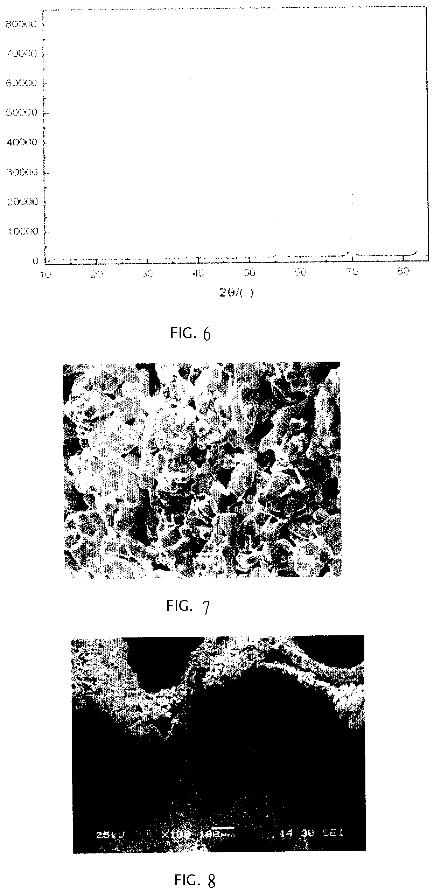

With reference to Figs. 6 to 8, it is shown that the porous tantalum material made in Example 3 has a foam structure with three-dimensional interconnecting pores. The foam structure has a foam skeleton accumulated by the tantalum powder, and tantalum particles located in the foam skeleton, and sintered neck structures formed between 50% to 95% of the tantalum particles.

The density, porosity, pore diameter and other mechanical properties of the obtained porous tantalum were tested by standard test methods such as GB/T5163-2006, GB/T5249-1985, GB/T6886-2001 and the like. The porous tantalum has three-dimensional interconnecting pores and less than 0.5% of impurities. The interconnecting pores are well-distributed. The tested porous tantalum has 3.5g/cm³ of density, higher than 40% of porosity, 150µm of average pore diameter, 2.0GPa of elastic modulus, 35MPa of yield strength, 40MPa of compressive strength, 17.3% of the amount of plastic deformation, 65MPa of tensile strength and 14.7% of percentage elongation. According to the anti-bending test on a basis of metal bending strength, the microstructure of the porous tantalum has less than 45% of fracture rate of the sintered neck structure, and larger than 55% of fracture rate of the interior of the tantalum particles.

### Example 4:

A porous tantalum rod is provided, which comprises a fastening structure 1 with screw thread formed on one end of the porous tantalum rod; a ball head shaped structure 4 formed on the other end of the porous tantalum rod; a slot 2 formed on the end-face of the fastening structure 1 for installing and using the surgical tools; as shown in Fig. 3, a through hole 3 formed on the center of the porous tantalum rod.

The porous tantalum material of the porous tantalum rod has a foam structure with three-dimensional interconnecting pores, which is produced by foam impregnation. The foam structure has a foam skeleton, and tantalum particles accumulated on the foam skeleton, and multiple sintering neck structures formed between the tantalum particles.

Tantalum powder having less than 50µm of diameter and less than 0.1% of the oxygen content as a raw material was mixed with a polyvinyl alcohol solution as a binder solution to form tantalum slurry. The tantalum slurry was casted into a polyurethane foam body. The polyurethane foam body with the tantalum slurry was dried, degreased, vacuum sintered, vacuum annealed and treated with normal post-treatments to obtain a porous tantalum.

In the exemplary embodiment, the poly urethane foam body has 0.56-0.72mm of pore diameter, 0.025g/cm³ of density and 50°-80° of hardness;
The polyurethane foam body with the tantalum slurry was dried under 10⁻²-1Pa of vacuity to remove water.

The dried polyurethane foam body and polyvinyl alcohol are separated from the dried tantalum slurry at 400-800°C of the temperature under 10⁻⁴-10⁻³Pa of vacuity or in a protective environment of inert gas and under the temperature kept for 30-120 minutes.

The dried tantalum slurry was sintered at 2000-2200°C under 10⁻⁴-10⁻³Pa of vacuity and under the temperature kept for 1-5 hours. The argon or other alternative inert gas was employed as a protective gas when keeping the temperature during sintering process, to obtain a porous sintered body.

After sintered, the porous sintered body was annealed by keeping the temperature at 1000-1250°C for 1-4 hours under 10⁻⁴-10⁻³Pa of vacuity, and then treated with normal post-treatments to obtain a porous tantalum.

### Example 5:

Tantalum powder having less than 50µm of diameter and less than 0.1% of the oxygen content as a raw material was mixed with a polyvinyl alcohol solution as a binder solution to form tantalum slurry. The tantalum slurry was casted into a polyurethane foam body having 0.48-0.89mm of pore diameter, 0.015-0.035g/cm³ and 50°-80° of hardness. The polyurethane foam body with the tantalum slurry was dried, degreased, vacuum sintered, vacuum annealed and treated with normal post-treatments to obtain a porous tantalum.

In the exemplary embodiment, polyvinyl alcohol was dissolved in the distilled water under heat to form a 5 wt% polyvinyl alcohol solution. 7 weight parts of tantalum powder and 1 weight part of the 5 wt% polyvinyl alcohol solution were mixed homogeneously and agitated to form pasty tantalum slurry. The polyurethane foam body was impregnated repeatedly until the pores of the polyurethane foam body were filled with the tantalum slurry;
the polyurethane foam body with the tantalum slurry was dried at 60-100°C for 4-8 hours under 1Pa of vacuity to remove water;
the dried polyurethane foam body were put into a tungsten device in a non-oxidizing atmosphere furnace which was heated up to 800°C at a proper temperature increasing rate. The dried organic foam body was degreased under argon having at least 99.999% of purity as a protective gas. The pure argon was introduced in to the furnace as a protective gas for 30 minutes before increasing the temperature to exclude the air in the furnace. The temperature was increased from room temperature to 400°C at a rate of 1°C/min with argon flowing at a rate of 0.5L/min and kept for 30 minutes, and then increased from 400 to 800°C at a rate of 0.5°C/min with argon flowing at a rate of 1L/min and kept for 120 minutes. Then, the power was turned off and the degreased sample was cooled down with the temperature in the furnace while argon flowing at a rate of 1L/min. The argon supplier was shut off until the temperature of the degreased sample was decreased to the room temperature;
the degreased sample in the tungsten device was heated in a sintering furnace and sintered by increasing the temperature to 2200°C at a proper rate. The vacuity reached to 10⁻⁴Pa before increasing the temperature in the sintering furnace. The temperature in the sintered furnace was increased from room temperature to 1200°C at a rate of 10-15°C/min and kept for 30 minutes under 10⁻⁴Pa of vacuity, and then increased to 1500°C at a rate of 10°C/min and kept for 30min under 10⁻⁴-10⁻³Pa of vacuity, and then increased to 2200°C at a rate of 6°C/min and kept for 120 minutes under 10⁻³Pa of vacuity. After sintered, under 10⁻³ Pa of vacuity, the temperature was decreased to 1600°C at a rate of 10-15°C/min and kept for 30 minutes, and then decreased to 1200°C at a rate of 12°C/min and kept for 60min, and then decreased to 800°C at a rate of 10°C/min. Then, the sintered sample was cooled naturally;
the sintered sample was put into the corundum container in an annealing furnace under proper vacuity and annealed by increasing the temperature to 1250°C at a proper rate. The vacuity reached to 10⁻⁴Pa before increasing the temperature in the annealing furnace. The temperature in the annealing furnace was increased from room temperature to 1250°C at a rate of 15°C/min and kept 240 minutes under 10⁻⁴-10⁻³Pa of vacuity, and then decreased to 1000°C at a rate of 5°C/min and kept for 180min under 10⁻⁴-10⁻³Pa of vacuity, and then decreased to 800°C at a rate of 10°C/min and kept for 120 minutes under 10⁻⁴Pa of vacuity, and then decreased to room temperature at a rate of 20°C/min under 10⁻⁴Pa of vacuity.

At last, the annealed sample was treated with normal post-treatments and a porous tantalum rod described in Example 1 was obtained.

## Claims

1. A porous tantalum rod comprising a fastening structure formed on one end of the porous tantalum rod, **characterized in that**, a through hole formed on the center of the porous tantalum rod; and the porous tantalum material used on the porous tantalum rod produced by foam impregnation and having a foam structure with three-dimensional interconnecting pores, wherein the foam structure has a foam skeleton, tantalum particles accumulated on the foam skeleton, and multiple sintering neck structures formed between the tantalum particles, wherein the porous tantalum material is produced by sintering the tantalum powder having an average diameter of less than 50µm and oxygen content less than 0.1%, wherein the produced porous tantalum material has 30-85% of porosity and 150-600µm of pore diameter, and the sintering neck structures are formed between at least 50%-95% of the tantalum particles.

2. The porous tantalum rod as claimed in claim 1, wherein the fastening structure is a screwed structure, and having a slot formed on the end-face of the screwed structure.

3. The porous tantalum rod as claimed in claim 1 or 2, wherein the other end of the porous tantalum rod is ball head shaped.

4. The porous tantalum rod as claimed in claim 1, 2 or 3,_the porous tantalum material is prepared via steps of:
(a) providing an organic binder, dispersant and tantalum powder;
(b) mixing the organic binder and the dispersant to form a solution and then mixing the solution and the tantalum powder to form tantalum slurry;
(c) providing an organic foam body, wherein the organic foam body has multiple pores;
(d) casting the tantalum slurry into the organic foam body and impregnating the casted organic foam body with the tantalum slurry until the pores of the organic foam body are filled with the tantalum slurry;
(e) drying the impregnated organic foam body with the tantalum slurry to remove the dispersant;
(f) degreasing the dried organic foam body to separate the dried tantalum slurry from the organic binder and the organic foam body in a protective environment of inert gas;
(g) vacuum sintering the dried tantalum slurry to obtain a porous sintered body, wherein the porous sintered body has a foam skeleton, sintered tantalum particles located on the foam skeleton, and multiple sintering neck structures formed between the tantalum particles; and
(h) vacuum annealing and treating the porous sintered body with normal post-treatments to obtain the porous tantalum.

5. The porous tantalum rod as claimed in claim 4, wherein the organic foam body in the preparation of the porous tantalum material is a polyurethane foam body having 0.48-0.89mm of pore diameter, 0.015-0.065g/cm3 of density and larger than 40° of hardness.

## Patentansprüche

1. Poröser Tantalstab, umfassend eine Befestigungsstruktur, die an einem Ende des porösen Tantalstabs gebildet ist, **dadurch gekennzeichnet, dass** ein Durchgangsloch in der Mitte des porösen Tantalstabs gebildet ist; und das poröse Tantalmaterial, das für den porösen Tantalstab verwendet wird, durch Schaumimprägnierung produziert wird und eine Schaumstruktur mit dreidimensionalen, mit einander verbundenen Poren aufweist, wobei die Schaumstruktur ein Schaumskelett, Tantalpartikel, die an dem Schaumskelett kumuliert sind, und mehrere Sinterhalsstrukturen, die zwischen den Tantalpartikeln gebildet sind, aufweist, wobei das poröse Tantalmaterial durch Sintern des Tantalpulvers mit einem mittleren Durchmesser von weniger als 50 µm und einem Sauerstoffgehalt von weniger als 0,1 % produziert wird, wobei das produzierte poröse Tantalmaterial eine Porosität von 30-85 % und einen Porendurchmesser von 150-600 µm aufweist und die Sinterhalsstrukturen zwischen zumindest 50 %-95 % der Tantalpartikel gebildet sind.

2. Poröser Tantalstab nach Anspruch 1, wobei die Befestigungsstruktur eine geschraubte Struktur ist und einen Schlitz aufweist, der an der Endfläche der geschraubten Struktur gebildet ist.

3. Poröser Tantalstab nach Anspruch 1 oder 2, wobei das andere Ende des porösen Tantalstabs kugelkopfförmig ist.

4. Poröser Tantalstab nach Anspruch 1, 2 oder 3, wobei das poröse Tantalmaterial durch die folgenden Schritte hergestellt wird:
(a) ein Bereitstellen eines organischen Bindemittels, eines Dispergiermittels und eines Tantalpulvers;
(b) ein Mischen des Bindemittels und des Dispergiermittels, um eine Lösung zu bilden, und dann ein Mischen der Lösung und des Tantalpulvers, um eine Tantalschlämme zu bilden;
(c) ein Bereitstellen eines Körpers aus organischem Schaum, wobei der Körper aus organischem Schaum mehrere Poren aufweist;
(d) ein Gießen der Tantalschlämme in den Körper aus organischem Schaum und ein Imprägnieren des gegossenen Körpers aus organischem Schaum mit der Tantalschlämme, bis die Poren des Körpers aus organischem Schaum mit der Tantalschlämme gefüllt sind;
(e) ein Trocknen, um das Dispergiermittel zu entfernen, des imprägnierten Körpers aus organischem Schaum mit der Tantalschlämme;
(f) ein Entfetten des getrockneten Körpers aus organischem Schaum in einer Schutzumgebung aus Inertgas, um die getrocknete Tantalschlämme von dem organischen Bindemittel und dem Körper aus organischem Schaum zu trennen;
(g) ein Vakuumsintern der getrockneten Tantalschlämme, um einen porösen gesinterten Körper zu erhalten, wobei der poröse gesinterte Körper ein Schaumskelett, gesinterte Tantalpartikeln, die sich an dem Schaumskelett befinden, und mehrere Sinterhalsstrukturen, die zwischen den Tantalpartikeln gebildet sind, aufweist; und
(h) ein Vakuumglühen und Behandeln des porösen gesinterten Körpers mit normalen Nachbehandlungen, um das poröse Tantal zu erhalten.

5. Poröser Tantalstab nach Anspruch 4, wobei der Körper aus organischem Schaum bei der Herstellung des porösen Tantalmaterials ein Polyurethanschaum mit einem Porendurchmesser von 0,48-0,89 mm, einer Dichte von 0,015-0,065 g/cm3 und einer Härte von mehr als 40° ist.

## Revendications

1. Tige poreuse en tantale comprenant une structure de fixation formée sur une extrémité de la tige poreuse en tantale, **caractérisée en ce qu'**un trou traversant est formé au centre de la tige poreuse en tantale ; et le matériau de tantale poreux utilisé sur la tige poreuse en tantale est produit par imprégnation de mousse et a une structure de mousse dotée de pores tridimensionnels interconnectés, ladite structure de mousse possédant un squelette en mousse, des particules de tantale accumulées sur le squelette en mousse, et de multiples structures de cols de frittage formées entre les particules de tantale, ledit matériau de tantale poreux étant produit par frittage de la poudre de tantale qui présente un diamètre moyen inférieur à 50 µm et une teneur en oxygène inférieure à 0,1 %, ledit matériau de tantale poreux produit présentant 30 à 85 % de porosité et 150 à 600 µm de diamètre de pore, et les structures de cols de frittage étant formées entre au moins 50 % à 95 % des particules de tantale.

2. Tige poreuse en tantale selon la revendication 1, ladite structure de fixation étant une structure vissée et comportant une rainure formée sur la face d'extrémité de la structure vissée.

3. Tige poreuse en tantale selon la revendication 1 ou 2, l'autre extrémité de la tige poreuse en tantale étant en forme de tête sphérique.

4. Tige poreuse en tantale selon la revendication 1, 2 ou 3, le matériau de tantale poreux étant préparé par les étapes de :
(a) fourniture d'un liant organique, d'un dispersant et d'une poudre de tantale ;
(b) mélange du liant organique et du dispersant pour former une solution puis mélange de la solution et de la poudre de tantale pour former une suspension de tantale ;
(c) fourniture d'un corps de mousse organique, ledit corps de mousse organique comportant de multiples pores ;
(d) coulage de la suspension de tantale dans le corps de mousse organique et imprégnation du corps de mousse organique ayant subi le coulage avec la pâte de tantale jusqu'à ce que les pores du corps de mousse organique soient remplis par la suspension de tantale ;
(e) séchage du corps de mousse organique imprégné avec la suspension de tantale pour enlever le dispersant ;
(f) dégraissage du corps de mousse organique séché dans un environnement protecteur de gaz inerte pour séparer la suspension de tantale séchée du liant organique et du corps de mousse organique;
(g) frittage sous vide de la suspension de tantale séchée pour obtenir un corps fritté poreux, ledit corps fritté poreux possédant un squelette en mousse, des particules de tantale frittées situées sur le squelette en mousse, et de multiples structures de cols de frittage formées entre les particules de tantale ; et
(h) recuit sous vide et traitement du corps fritté poreux avec des post-traitements normaux pour obtenir le tantale poreux.

5. Tige poreuse en tantale selon la revendication 4, ledit corps de mousse organique dans la préparation du matériau de tantale poreux étant un corps de mousse polyuréthane présentant 0,48 à 0,89 mm de diamètre de pore, 0,015 à 0,065 g/cm3 de masse volumique et plus de 40° de dureté.
